# EUROPEAN PATENT APPLICATION

(11) **EP 0 544 349 A1**
(43) Date of publication of application: **02.06.1993**
(21) Application number: 92203396.4
(22) Date of filing: 05.11.1992
(51) Int. Cl.: A23L 1/275

(54) **Liquid with coloured particles**

(30) Priority: 12.11.1991 EP 91202936
(71) Applicant: UNILEVER N.V., NL-3000 DK Rotterdam (NL); UNILEVER PLC, London EC4P 4BQ (GB)
(72) Inventor: Slemmer, Alfred Daniel, NL-2623 BC Delft (NL); Visser, Cornelis, NL-2636 CA Schipluiden (NL)
(74) Representative: Sikken, Antonius H. J. M.

(57) **Abstract**

A coloured carrier material for liquids comprises an at least partially gelatinized starch based matrix material and a colouring agent. The liquids are preferably transparent and edible, such as dressings. A process of preparing the coloured carrier material has also been described.

## Description

The present invention relates to liquids containing coloured particles as well as to a process for preparing such liquids.

Transparent edible liquids with pieces of solid coloured material of vegetable origin are known as the so-called French salad dressings or vinaigrette type products. Usually such products comprise a blend of water, vinegar, sweeteners and a gum or gum system that promotes a stable suspension of the coloured, solid particles of vegetables, herbs and spices. In order to keep the flavour-producing herbs and spices permanently suspended in the liquid strict requirements to the suspending medium should be observed.

In other areas of technology, liquids should sometimes temporarily carry a marking material. For this purpose dyes are dissolved in the liquids but this is not always an acceptable solution, since the dye migrates and may contaminate e.g. machine parts, like pumps, and usually cannot be removed from the liquid. In such cases a particulate coloured marker material, which by simple filtration can be removed from the liquid would be of advantage.

In the area of detergents, shampoos, and the like material, it is sometimes of advantage that small coloured particles are present in the product to give it a fancy appearance. When rinsing out the shampoo after hair washing the particles may remain as funny speckles attached to the hair. The coloured particles may additionally serve to introduce active ingredients into the formulation.

### SUMMARY OF THE INVENTION

For the purpose of preparing speckled liquids which meet the above requirements, it has been found that a matrix which is based on an at least partially gelatinized starch or starch comprising material, like flours and in which matrix a colour agent is incorporated is a particularly useful material.

Therefore, the present invention relates to liquids containing a particulate coloured carrier material or marker material, which material comprises an at least partially gelatinized starch based matrix material which contains a colouring agent.

### DETAILS OF THE INVENTION

Preferably the liquid, the colouring agent and the matrix material are either food products or suited to be used in food products.
The liquid preferably is a transparent liquid, such as a vinaigrette.

US patent 4,112,125 describes foodstuffs with coloured particles of pre-gelatinized starch, but these are solid foodstuffs, in particular dough products like muffins, in which relatively coarse coloured particles have been incorporated.

According to the present invention the specific gravity of such particles can also be varied, with the effect of a larger number of degrees of freedom for the formulation of the suspending medium. It is a great advantage if, moreover, such particulate material could contribute organoleptic properties beyond one flavour or one taste. The coloured particles present in the liquid, may absorb the liquid and swell and in doing so their buoyancy properties adjust themselves to the particular liquid. The extent of swelling is related to the composition of the matrix material and depends on the degree of gelatinization. The matrix material should in such cases be swellable, but essentially not soluble in the liquid in which they are applied. The term "liquids" is to be understood throughout this specification and the attached claims as comprising regular liquids, including dispersions, slurries and purees, as well as semi-liquids like viscous, pasty and spreadable materials.

The invention encompasses also dry compositions which by the addition of water can be reconstituted to a liquid and which compositions comprise an effective amount of particulate coloured carrier material comprising a colouring agent in an at least partially gelatinized starch based matrix material.
In particular the dry compositions are taken from the group comprising instant soup mixtures, instant sauce mixtures, instant dressing mixtures and instant mayonnaise mixtures.

The matrix material of the coloured carrier material according to the present invention is selected from the group of the following at least partially gelatinized starches: cereal starches, root starches, tubular starches, materials comprising these starches, such as flours, and mixtures thereof.
Examples of cereal starches are rice starch, wheat starch, rye starch, oat starch, maize or corn starch; an example of a tubular starch is potato starch and an example of a root starch is tapioca starch. Also mixtures of these various starches or starch comprising materials, such as various flours, may be used.

Other at least partially gelatinized starches are the waxy starches, like waxy maize starch or waxy rice starch, and mixtures of waxy starches and normal starches. Also amylose and starch derivatives, such as starch esters, starch ethers, dextrins, cyclodextrins, and heat-moisture treated starches may be used.

The starch may be subjected to gelatinisation in the presence of a colouring agent, but the starch material may also have been gelatinized, at least partially, when the starch material is coloured.

The coloured carrier material according to the present invention may also comprise an additive selected from the group consisting of acidulants, such as food grade acids, lemon juice, vinegar; wine; antioxidants, particularly those derived from natural materials, such as tocopherols; spice extracts, and the like; emulsifiers, such as fatty acid monoglycerides; flavouring agents, preferably savoury flavouring agents, but also wine flavours; flavour precursors; sweeteners; stabilizers, such as gums; vitamins; minerals, such as common salt; herbs; spices; vegetables; preservatives; egg material; proteins of vegetable, fungal or animal origin; and mixtures thereof.

The colouring agent preferably is an edible colouring agent and preferably it is a colouring agent from natural origin. Examples of suitable colouring agents are annatto extract, (red) beet powder, curcuma, caramel, grape skin extracts, carrot oil, paprika, turmeric, chlorophyll, carotene synthetic, preferably edible dyes, and mixtures thereof. By mixing certain colours various shades or even new colours may be obtained.
If the coloured carrier material is not applied in food, but rather as a marker material, then various types of synthetic dyes, particularly fluorescent dyes, may be applied. Differently coloured carrier materials may be mixed before drying.
The carrier material particles may be monochromatic and polychromatic. Also two or more different colouring agents may be used. For particular purposes it may be advantageous that these have different leaching or extracting characteristics, so that one or more colouring agents are gradually leached out from the particles by the liquid in which the particles are dispersed, whereas the other colouring agents are to a lesser extent or not at all leached out. This may give striking colour effects. The colouring agent may be dispersed throughout the particle or only attached on the surface.

The coloured carrier material or marker material may be brought into any desired form or shape, such as fancy forms, like animals, trees, letters; polygonal forms; multifacetted forms; triangular; squares; circular form; ellipsoids. The thickness of the carrier material may vary. The required particle size of the coloured carrier material may be adjusted by comminution and sieving techniques.

The coloured carrier material is dispersed in the liquid in particulate form. The size and the concentration of the particles are preferably chosen such that they can be distinguished separately by the naked eye.
The liquid medium may be an edible material, particularly a foodstuff, and preferably is a transparent liquid. Examples of such transparent products are the French salad dressings or vinaigrettes, which may be oil-free products. These products comprise water, vinegar, dextrose, salt, flavourings and spices, lemon juice, gums and stabilizers, vegetable particles, emulsifiers, and mixtures thereof. The coloured carrier material particles may also be applied in soups, mayonnaise, ketchups, dressings (like salad dressings), sauces, spreads, peanut butter, margarine, ice cream and ice cream based products, sorbets, jams, apple sauce, and similar products, which may obtain an attractive appearance by the added speckles.

The coloured carrier materials may be produced in such a way that they have an expanded texture, which gives them a different specific gravity and which enables a better liquid absorption. Also further ingredients, such as flavours, may be incorporated into the coloured carrier material with the purpose that these are gradually leached out by the liquid medium. In this way specific flavours, which cannot be applied otherwise can be imparted to the liquid, in combination with the presence of visible particles. When the coloured carrier material particles is applied in admixture with usual ingredients such as vegetables, herbs and/or spices, the attractive visible appearance of for example a dressing product is retained, while flavouring and/or colouring properties are added. An additional advantage of the coloured carrier or marker material particles is that they are highly thermostable, so that they can be heated to the relatively high temperatures used during food preparation without damage to their organoleptic and visual properties.

The coloured particles may, according to the invention, advantageously be used for preparing speckled non-food liquids, such as detergents, shampoos and industrial liquids.

A process for preparing a coloured carrier material for use in the present invention comprises the following steps:
(a) mixing a colouring agent into a slurry or dough of a starch based matrix material and a liquid,
(b) heating the coloured mixture to at least partial gelatinisation and
(c) drying the coloured and at least partially gelatinised starch based mixture.

The coloured and at least partially gelatinised starch based material preferably is shaped into the required form between steps (b) and (c) of the said process.

The gelatinization is preferably effected by heating the mixture of the matrix material, the liquid, the colouring agent and, optionally, further additives, which mixture is confined between two parallel surfaces of glass, metal or other heat-resistant material in the presence of moisture in the mixture.
Under certain conditions it is also possible to use one surface as the bottom plate. When the starch material of the matrix material is a native starch, the use of two parallel surfaces is almost mandatory, but if the starch material of the matrix material consists of 25-100% by weight of pregelatinized starch, the balance being native starch, then it is possible to use one surface only in the temperature range 100-150°C. The distance between the surfaces generally is about 0.1-2 mm or more. The surfaces are e.g. plates and travelling belts.

Alternatively, the gelatinization and the shaping of the carrier material is effected in an extrusion device. If the extruder operates under vacuum, the product upon extrusion will expand to a porous structure. Such an expanded product is also obtained by incorporating a blowing agent, like a mixture of alkali metal or ammonium bicarbonate and an innocuous acid, or by a proper selection of the starch materials of the matrix material.

In another embodiment of the present invention the colouring agent is mixed with a slurry of a starch based matrix material which is at least partially pregelatinized and an edible liquid such as water, and then the mixture is dried. The gelatinization step can be deleted if the matrix material is a totally pregelatinized starch.

The present invention particularly relates to transparent dressings, which are characterized in that they comprise 0.1-15% by weight, based on the total dressing composition, of particles of coloured carrier material
comprising an at least partially gelatinized starch based matrix material and a colouring agent.

The present invention also relates to a process for preparing a liquid containing coloured carrier material particles, which process is characterized in that the above described particles of the coloured carrier material according to the present invention are incorporated into a suitable liquid which may or may not be a foodstuff.

When the process relates to the colouring of foodstuffs, the invention comprises that particles of the coloured carrier material are incorporated into a liquid foodstuff. Preferably the foodstuffs are selected from the group consisting of: soups, mayonnaise, dressings, ketchups, sauces, margarine, spreads, peanut butter, ice cream, sorbets, jams, and fruit pulps, like apple sauce.
The present invention also relates to a process for colouring rehydratable materials, which is characterized in that an effective amount of particles of the coloured carrier material according to the present invention is incorporated into a rehydratable material. Preferably the rehydratable material is a food composition comprising instant soup mixtures, instant sauce mixtures, instant dressing mixtures or instant mayonnaise mixtures.

Finally, the present invention relates to a process of preparing transparent dressings comprising coloured particles of carrier material particles, which is characterized in that particles of the coloured carrier material comprising an at least partially gelatinized starch based matrix material and a colouring agent.

The invention will now be illustrated by the following examples:

### EXAMPLE I

A clear aqueous phase was formed by dissolving 5 grams of Keltrol RD (Trade Mark; a readily dispersible xanthan gum, ex Kelco International, UK) in 600 grams of water. In order to preserve the clear solution an amount of 0.2% by weight on the total composition of potassium sorbate were added. 1 gram of chlorophyll powder was stirred into a mixture of 10 grams of pregelatinized waxy maize starch and 30 grams of waxy maize starch in 100 grams of water.
The green coloured slurry obtained was spread in a thin layer of about 1 mm thickness sandwiched between two glass plates. The mixture was subjected to gelatinization by heating it for 45 minutes at 100°C. Subsequently, the top glass plate was removed and the gelatinized coloured starch material was dried for 30 minutes at 100°C.
The dried material could easily be removed from the glass plate in the form of about 0.1-0.2 mm thick flake-like deep green coloured material. The flakes were reduced to a particle size of about 1-5 mm and 1% by weight (based on the weight of the aqueous phase as prepared above) of these deep green particles were stirred into the clear aqueous phase. A pleasant looking vinaigrette-like solution was obtained, in which the coloured particles remained in buoyant conditions.

### EXAMPLE II

The same deep green coloured gelatinized starch material as prepared in Example I was stirred into Yogonaise (a yoghurt based dressing material ex Calvé, The Netherlands) in an amount of 0.2% by weight, based on the yogonaise. A very pleasant looking product was obtained, in which the clear, deep green particles contrasted excellently against the white background of the dressing.

### EXAMPLE III

A mixture of 10 grams of pregelatinized waxy maize starch, 30 grams of native wheat starch, 10 grams of yeast extract powder and 5 grams of curcuma powder was suspended in 100 grams of water and the obtained yellow slurry was spread in a thin layer of about 1 mm thickness on a glass plate. The mixture was gelatinized and dried by heating it for 60 minutes at 100°C.

The obtained clear yellow flake-like material was taken off the glass plate and reduced in size to pieces of average size between 1-5 mm. These pieces were stirred into tomato puree in an amount of 0.2% by weight. A yellow speckled tomato puree was obtained, in which the coloured pieces had a pleasant savoury taste.

### EXAMPLE IV

In the same way as described in Example I and using the same materials, except that instead of chlorophyll powder, now 5 grams of red beet powder were used, a red coloured material was obtained. This red coloured gelatinized starch material was stirred into yogonaise as described in Example II. A very pleasant looking product was obtained in which the deep red particles contrasted vividly against the white background of the dressing.

## Claims

1. A liquid, characterized in that it comprises an effective amount of particulate coloured carrier material comprising an at least partially gelatinized starch based matrix material and a colouring agent.

2. A liquid according to claim 1, characterized in that the coloured carrier material has a form or shape selected from the group consisting of: fancy forms, polygonal forms, multifacetted forms, triangular form, square form, circular form, and ellipsoid form.

3. A liquid according to claim 1 or 2, which is a foodstuff.

4. A liquid foodstuff according to claim 3, characterized in that the coloured carrier material comprises an additive selected from the group consisting of: acidulants, antioxidants, emulsifiers, flavouring agents, flavouring precursors, sweeteners, stabilizers, vitamins, minerals, herbs, spices, vegetables, preservatives, egg material, proteins of vegetable, fungal or animal origin, and mixtures thereof.

5. A liquid foodstuff according to claim 3 or 4, characterized in that the foodstuff is selected from the group consisting of: soups, mayonnaise, dressings, ketchups, sauces, margarine, spreads, peanut butter, ice cream, sorbets, jams, and fruit pulps.

6. A liquid foodstuff according to claim 5, characterized in that it is a transparent dressing which comprises from 0.1 to 15% by weight, based on the total dressing composition, of coloured carrier material.

7. A rehydratable solid material, characterized in that it comprises an effective amount of particles of coloured carrier material comprising an at least partially gelatinized starch based matrix material and a colouring agent.

8. A rehydratable solid material according to claim 7, characterised in that the rehydratable solid material is selected from the group consisting of: instant soup mixtures, instant sauce mixtures, instant dressing mixtures or instant mayonnaise mixtures.

9. A process of preparing a liquid foodstuff containing coloured carrier material particles, characterized in that particles of coloured carrier material comprising an at least partially gelatinized starch based matrix material and a colouring agent are incorporated into a liquid foodstuff.

10. A process of preparing a rehydratable solid material, characterized in that an effective amount of particles of coloured carrier material comprising an at least partially gelatinized starch based matrix material and a colouring agent is incorporated into a rehydratable solid material.
